# EUROPEAN PATENT SPECIFICATION

(11) **EP 2 892 917 B1**
(45) Date of publication and mention of the grant of the patent: **23.08.2017**
(21) Application number: 13759505.4
(22) Date of filing: 06.09.2013
(51) Int. Cl.: C07K 14/435, A61K 35/741, A61K 38/17, A61K 35/00, A61K 35/74, A61K 38/57

(54) **PROBIOTIC BACTERIA AS CARRIER FOR A HELMINTH-DERIVED IMMUNOMODULATOR FOR THE TREATMENT OF INFLAMMATORY DISORDERS**
PROBIOTISCHES BAKTERIUM ALS TRÄGER FÜR EINEN AUS ENTHELMINTHEN STAMMENDEN IMMUNMODULATOR ZUR BEHANDLUNG VON ENTZÜNDLICHEN KRANKHEITEN
BACTÉRIES PROBIOTIQUES SERVANT DE SUPPORT D'UN IMMUNOMODULATEUR DÉRIVÉ D'HELMINTHE POUR LE TRAITEMENT DES TROUBLES INFLAMMATOIRES

(30) Priority: 06.09.2012 EP 12183268
(43) Date of publication of application: 15.07.2015
(73) Proprietor: Humboldt-Universität zu Berlin, 10099 Berlin (DE); Freie Universität Berlin, 14195 Berlin (DE)
(72) Inventor: HARTMANN, Susanne, 12307 Berlin (DE); WIELER, Lothar, 14109 Berlin (DE); WHELAN, Rose, 10827 Berlin (DE); RAUSCH, Sebastian, 10997 Berlin (DE)
(74) Representative: Gulde & Partner
(86) International application number: PCT/EP2013/068472
(87) International publication number: WO 2014/037505

(56) References cited:
- WO-A1-2011/086172
- WO-A2-02/056900
- WO-A2-2008/015014
- WESTENDORF A M ET AL: "Intestinal immunity of Escherichia coli NISSLE 1917: a safe carrier for therapeutic molecules", FEMS IMMUNOLOGY AND MEDICAL MICROBIOLOGY, ELSEVIER SCIENCE B.V., AMSTERDAM, NL, vol. 43, no. 3, 1 March 2005 (2005-03-01), pages 373-384, XP027804149, ISSN: 0928-8244 [retrieved on 2005-03-01]
- H. J. CHOI ET AL: "Enhanced Wound Healing by Recombinant Escherichia coli Nissle 1917 via Human Epidermal Growth Factor Receptor in Human Intestinal Epithelial Cells: Therapeutic Implication Using Recombinant Probiotics", INFECTION AND IMMUNITY, vol. 80, no. 3, 19 December 2011 (2011-12-19), pages 1079-1087, XP055050694, ISSN: 0019-9567, DOI: 10.1128/IAI.05820-11
- SYA N. UKENA ET AL: "Probiotic Escherichia coli Nissle 1917 Inhibits Leaky Gut by Enhancing Mucosal Integrity", PLOS ONE, vol. 2, no. 12, 12 December 2007 (2007-12-12), page e1308, XP055082936, ISSN: 1932-6203, DOI: 10.1371/journal.pone.0001308
- SILVIA C. RESTA-LENERT ET AL: "Modulation of Intestinal Barrier Properties by Probiotics: Role in Reversing Colitis", ANNALS OF THE NEW YORK ACADEMY OF SCIENCES, vol. 1165, no. 1, 28 May 2009 (2009-05-28) , pages 175-182, XP055082975, ISSN: 0077-8923, DOI: 10.1111/j.1749-6632.2009.04042.x
- RUDOLF MENNIGEN ET AL: "Effect of Probiotics on Intestinal Barrier Function", ANNALS OF THE NEW YORK ACADEMY OF SCIENCES, vol. 1165, no. 1, 28 May 2009 (2009-05-28) , pages 183-189, XP055083153, ISSN: 0077-8923, DOI: 10.1111/j.1749-6632.2009.04059.x
- STEIDLER L ET AL: "Treatment of murine colitis by Lactococcus lactis secreting interleukin-10", SCIENCE, AMERICAN ASSOCIATION FOR THE ADVANCEMENT OF SCIENCE, WASHINGTON, DC; US, vol. 289, no. 5483, 25 August 2000 (2000-08-25), pages 1352-1355, XP002208404, ISSN: 0036-8075, DOI: 10.1126/SCIENCE.289.5483.1352
- STEIDLER L: "GENETICALLY ENGINEERED PROBIOTICS", BAILLIERE'S BEST PRACTICE AND RESEARCH. CLINICAL GASTROENTEROLOGY, BAILLIERE TINDALL, LONDON, US, vol. 17, no. 5, 1 January 2003 (2003-01-01), pages 861-876, XP009028993, ISSN: 1521-6918, DOI: 10.1016/S1521-6918(03)00072-6
- YUVARAJ SARAVANAN ET AL: "Transgenic probiotica as drug delivery systems: the golden bullet?", EXPERT OPINION ON DRUG DELIVERY, INFORMA HEALTHCARE, GB, vol. 4, no. 1, 1 January 2007 (2007-01-01) , pages 1-3, XP008095174, ISSN: 1742-5247, DOI: 10.1517/17425247.4.1.1
- MARCO M L ET AL: "Towards understanding molecular modes of probiotic action", CURRENT OPINION IN BIOTECHNOLOGY, LONDON, GB, vol. 17, no. 2, 1 April 2006 (2006-04-01), pages 204-210, XP024962878, ISSN: 0958-1669 [retrieved on 2006-04-01]
- J.-R. LIU ET AL: "Expression of Rumen Microbial Fibrolytic Enzyme Genes in Probiotic Lactobacillus reuteri", APPLIED AND ENVIRONMENTAL MICROBIOLOGY, vol. 71, no. 11, 1 November 2005 (2005-11-01), pages 6769-6775, XP55083127, ISSN: 0099-2240, DOI: 10.1128/AEM.71.11.6769-6775.2005
- SCHIERACK PETER ET AL: "Parasite-specific immunomodulatory functions of filarial cystatin", INFECTION AND IMMUNITY, AMERICAN SOCIETY FOR MACROBIOLOGY, USA, vol. 71, no. 5, 1 May 2003 (2003-05-01), pages 2422-2429, XP002413961, ISSN: 0019-9567, DOI: 10.1128/IAI.71.5.2422-2429.2003
- HARTMANN S ET AL: "Modulation of host immune responses by nematode cystatins", INTERNATIONAL JOURNAL OF PARASITOLOGY, PERGAMON PRESS, GB, vol. 33, no. 11, 1 January 2003 (2003-01-01), pages 1291-1302, XP002413960, ISSN: 0020-7519, DOI: 10.1016/S0020-7519(03)00163-2

## Description

About 3.6 million Europeans and US-Americans suffer from Crohn's disease (CD) or ulcerative colitis (UC), collectively known as inflammatory bowel diseases (IBD). Both CD and UC are chronic inflammatory disorders characterized by periods of remission and relapse with subsequently unpredictable fluctuations in symptom severity. Symptoms can occur at any age, but the onset generally appears between the ages of 15 and 35 and can then severely affect the quality of life for patients. Symptoms include permanent diarrhoea, cramping, abdominal pain, weight loss, fatigue and rectal bleeding during disease flare-ups. Common complications, especially in CD patients, are formation of deep ulcers with high risk of intestinal ruptures, or intestinal blockade due to swelling and scar tissue. Areas other than the intestine such as the joints, skin, eyes and liver can also be affected (http://www.cdc.gov/ibd/).

In IBD, the immune system mistakes bacterial components, food or other materials in the intestine for foreign substances and finally attacks the cells of the intestine. Many potential factors are involved in the induction and propagation of IBD, such as inheritance of gene loci linked to IBD susceptibility and life style associated factors, such as diet and smoking. The intestinal bacterial flora is central for the initiation of IBD development, but eventually activation of the adaptive immune system leads to the strong production of inflammatory cytokines by T helper cells, which constitute the dominant force driving chronic inflammation.

A wide range of therapies are used to treat IBD symptoms as the numerous factors involved in the initiation and propagation of IBD mean that response to specific treatments varies greatly between individual patients. Antibiotic treatment may temporarily improve the symptoms by reducing the gastro-intestinal bacterial load, but may also lead to malnourishment or exacerbate the condition by allowing resistant pathogens to flourish in the less competitive environment. Patients are routinely treated with corticosteroids, which help reduce inflammation. Corticosteroid treatment can be highly effective when administered long term, however it also results in the highest adverse effect incidences. Up to 50% of patients treated with corticosteroids develop side effects ranging from metabolic disorders such as glucose intolerance, malnourishment, dermatological and mood/sleep disturbances, to osteoporosis, and finally resistance to treatment with long term usage. Corticosteroid sparing therapies, such as treatment with immunosuppressants are routinely administered in order to decrease the risk of side effects; however efficacy may also be decreased. The use of a steroid-sparing agent can induce remission in CD patients and when administered for prolonged periods can even provide long-term maintenance of remission. However, monoclonal antibody treatments (e. g. Infliximab targeting the inflammatory cytokine TNF-alpha) are very cost-intensive, especially when the need for long-term treatment is considered and TNF-alpha inhibition therapy can result in serious side effects.

The application of probiotic microorganisms has been proposed to have beneficial effects alone or in conjunction with conventional treatments in IBD patients. A summary of current probiotic based treatment approaches is provided by Mack et al. (Nutrients 2011, 3, 245-24). While application of *Lactococcus lactis* alone was insufficient for treatment of human IBD, transgenically modified *L. lactis* strains expressing immunosuppressants such as interleukin-(IL-) 10 and anti-TNF-alpha ameliorated colitis in mouse models. However, a recent metaanalysis of human IL-10 treatment has determined no significant effect in IBD patients compared to the placebo control.

Clearly the current treatments are less than ideal as they are often accompanied by severe side effects and while they may help to control symptoms and maintain remission, they offer no cure to the disease. Additionally many patients are refractory or, over time, respond insufficiently to the available medications. Thus, two thirds to three quarters of patients will at some point in their lives require medical surgery to remove heavily inflamed parts of the intestine or even the entire colon (http://www.cdc.gov/ibd/). Both forms of IBD thus generally affect patients for life and new treatments are urgently needed.

According to the present invention, a transgenic probiotic microorganism as defined in the claims is provided which is genetically engineered to express and secrete nematode derived cystatin.

The present invention is directed to a transgenic probiotic microorganism genetically engineered to express and secrete nematode derived cystatin, wherein the probiotic microorganism is *Escherichia coli* strain Nissle 1917; and wherein the nematode derived cystatin comprises or consists of:
- an amino acid sequence with SEQ ID NO. 1, 2 or 3; or
- an amino acid sequence that is at least 70% identical, preferably at least 80% identical, more preferably at least 90% identical and most preferably at least 99% identical to an amino acid sequence with SEQ ID NO. 1, 2 or 3.

Recently, an effective immune modulator has been identified, a cystatin derived from parasitic nematode. Nematode derived cystatin efficiently suppresses host immune responses in order to maintain survival of the nematode in the host. Nematode derived cystatin down-regulates inflammation and therefore ameliorates inflammatory pathologies such as airway hyper-reactivity and gastro-intestinal inflammatory diseases like e.g. colitis *in vivo* in murine models, see WO 2008/015014 A2. Macrophages are the target cell of nematode derived cystatin, acquiring a regulatory phenotype when exposed to the recombinant protein (Klotz et al., "A helminth immunomodulator exploits host singaling events to regulate cytokine production in macrophages", PLoS pathogens 2011, 7(1)). Macrophages exposed to nematode cystatin were also found to suppress inflammation when transferred to mice with airway hyper reactivity or gastro-intestinal inflammatory diseases like e.g. colitis, independent of the recipient mice ever being exposed to the protein.

A new application method for nematode derived cystatin has been developed as it enables for constant, prolonged exposure of such cystatin to the inflamed gut. Previously, nematode derived cystatin was given in relatively large doses as an intra-peritoneal injection, which significantly suppressed inflammation in a murine colitis model (WO 2008/015014 A2). This application method is unrealistic in human gastro-intestinal inflammatory diseases like e.g. IBD. The creation of transgenic probiotic microorganisms capable of expressing a highly efficient immunomodulator of parasitic origin is a novel approach aiming to improve therapy of IBD and other gastro-intestinal inflammatory disorders. Administration of the transgenic probiotic microorganism allows for long term secretion of the cystatin immune modulator in the gut at the site of inflammation, without exposing the recipient to a parasitic infection. The transgenic probiotic microorganism also bypasses the need to apply large amounts of protein orally, in which maintenance of protein integrity through the digestive process is a concern or other more systemic routes. Therefore, this innovative therapy combines the inherent beneficial effects of probiotic microorganisms with the additional potential of the nematode derived cystatin in a safe and localized manner.

As a new, innovative approach for long term application of the highly efficient nematode derived cystatin and treatment of gastro-intestinal inflammatory diseases like e.g. IBD, a transgenic probiotic microorganism is provided which is capable of expressing and secreting the nematode derived immune modulator. This transgenic probiotic excellently combines the already known effects of probiotic microorganism *Echerichia coli* Nissle (EcN) and nematode derived cystatin in the treatment of gastro-intestinal inflammatory diseases like e.g. colitis in a surprisingly beneficial way, while allowing for a cost effective and efficient therapy directly at the site of inflammation. The benefits are not impeded by any adverse effects, as no significant changes to the host immune parameters or health status were observed when piglets (as a model of the human gastrointestinal and immune system) were treated continually with the transgenic probiotic over a two week period. This transgenic probiotic therefore provides an ideal therapy for gastro-intestinal inflammatory diseases like e.g. IBD in a market clearly lacking a safe, effective, cost efficient treatment for a morbid disease with rising incidence in the industrialized and developing world.

The present invention is directed to a probiotic microorganism.

The term "microorganism" is used herein in its art recognised meaning and refers in particular to organisms selected from bacteria, archaea, unicellular fungi like e.g. yeast, algae and unicellular protozoa. Preferably, the term "microorganism" is used to denote bacteria and unicellular yeast.

For the purpose of the present invention, the term "probiotic" refers to a microorganism capable of exerting a beneficial effect upon administration to a suitable subject or host. Upon proper administration, a probiotic microorganism is capable to survive in said subject or host for a considerable period of time. In particular, the probiotic microorganism of the invention refers to a vital microorganism which, upon proper administration to said subject or host, is capable of proliferation and/or colonization within said subject or host. The skilled person is well aware of microorganisms that can be used as probiotics in the sense of the present invention. There is also ample literature available describing probiotic microorganisms used for treatment of numerous diseases like e.g. gastro-intestinal inflammatory diseases. A recent overview on the state of the art in this field is given by Mack et al (2011). In particularly, the probiotic microorganism of the invention is *Escherichia coli* strain Nissle 1917 (EcN). *Escherichia coli* Nissle 1917 (EcN) was first applied in 1917 as a therapy for gastro-intestinal disease in soldiers and has been available for use in humans since (Nissle et al., Zeitschrift für klinische Medizin 1951, 2 (3-4); 68). Several recent trials have observed significant responses to treatment with the probiotic EcN in gastrointestinal disease in infants and children as well as when rectally administered during acute flare-ups of ulcerative colitis. The colonization properties in humans and lack of immune activation by the bacterium make EcN an excellent carrier for immunosuppressive factors for treatment of gastro-intestinal inflammatory diseases like e.g. IBD. Thus, *Eschericia coli* Nissle 1917 has proven significant beneficial effects as therapeutic for ulcerative colitis. EcN effectively colonizes the gut without inducing immune activation in the host and is, therefore, an excellent carrier for the nematode derived immune modulator cystatin.

According to the present invention the probiotic microorganism is transgenic in that it is genetically engineered to express and secrete nematode derived cystatin. Cystatins are proteins of a family of cysteine protease inhibitors which contain four conserved cysteine residues supposed to be involved in disulfide bond formation. Nematode derived cystatins can be cystatins that are identical in sequence to cystatins derived from or originally described for nematodes. Nematode derived cystatins exhibit significant binding to the surface marker CD36. A suitable assay for testing CD36 binding capabilities is described in WO 2008/015014 A2. Nematode derived cystatins show binding to CHO cells transfected to express CD36, whereas nematode derived cystatins show no significant binding to CHO cells lacking CD36 expression.

Nematode derived cystatin ameliorates inflammation in murine models of colitis when injected intra-peritoneally, see e.g. WO 2008/015014 A2. The effect is mediated by the induction of regulatory cell types and an anti-inflammatory cytokine milieu.

The nematode derived cystatin expressed and secreted by the transgenic probiotic microorganism of the invention comprises or consists of:
- an amino acid sequence with SEQ ID NO. 1, 2 or 3; and/or
- an amino acid sequence that is at least 70% identical, preferably at least 80% identical, more preferably at least 90% identical and most preferably at least 99% identical to an amino acid sequence with SEQ ID NO. 1, 2 or 3.

SEQ ID NO. 1 denotes the amino acid sequence *of Acanthocheilonema viteae* cystatin, also called AvCystatin, with the Gene bank accession No. L43053.
SEQ ID NO. 2 denotes the amino acid sequence of *Onchocerca volvulus* cystatin with the Gene bank accession No. M37105.
SEQ ID NO. 3 denotes the amino acid sequence of *Brugia malayi* cystatin with Gene bank accession No. AF 177193_1.

The nematode derived cystatin may also comprise or consist of an amino acid sequence that is at least 70% identical, preferably at least 80% identical, more preferably at least 90% identical and most preferably at least 99% identical to an amino acid sequence with SEQ ID NO. 1, 2 or 3. Preferably, such nematode derived cystatins contain the four conserved cysteine residues of the cystatin family and bind to CD36.

Preferably, the nematode derived cystatin expressed and secreted by the transgenic probiotic microorganism of the invention comprises or consists of:
- an amino acid sequence with SEQ ID NO. 1; and/or
- an amino acid sequence that is at least 70% identical, preferably at least 80% identical, more preferably at least 90% identical and most preferably at least 99% identical to an amino acid sequence with SEQ ID NO. 1. Preferably, nematode derived cystatins comprising or consisting of an amino acid sequence that is at least 70% identical, preferably at least 80% identical, more preferably at least 90% identical, and most preferably at least 99% identical to an amino acid sequence with SEQ ID NO. 1 contain the four conserved cysteine residues of the cystatin family and bind to CD36.

The transgenic probiotic microorganism of the invention is genetically engineered to express and secrete nematode derived cystatin. The term "transgenic probiotic microorganism" in the sense of the present invention refers to probiotic microorganism which itself have been genetically modified to express and secrete nematode derived cystatin or to progeny thereof which still expresses and secretes nematode derived cystatin.

Starting from the amino acid sequence of the nematode derived cystatin, the skilled person is well aware of how to produce transgenic probiotic microorganisms of the invention. In general terms such transgenic probiotic microorganisms may be produced by cloning a nucleic acid molecule encoding for the desired nematode derived cystatin amino acid sequence into a vector molecule which allows for expression and secretion of the expression product and to transform or transfect a probiotic microorganism with said vector molecule. The skilled person is well aware of the degeneracy of the genetic code and knows that there is more than one possibility to design a nucleic acid molecule encoding for a given amino acid sequence. The amino acid sequence of *Acanthocheilonema viteae* cystatin with SEQ ID NO. 1, also called AvCystatin, may be expressed using a nucleic acid molecule comprising or consisting of the nucleic acid sequence with SEQ ID NO. 4, see nucleic acid sequence of *Acanthocheilonema viteae* cystatin, also called AvCystatin, with the Gene bank accession No. L43053.

The transgenic probiotic microorganism may be stably or transiently transformed or transfected to express and secrete the desired nematode derived cystatin, preferably the transgenic probiotic microorganism is genetically engineered to stable express and secrete the desired nematode derived cystatin.

The present invention is also directed to a pharmaceutical composition comprising a transgenic probiotic microorganism of the invention and at least one pharmaceutically acceptable excipient.

The transgenic probiotic microorganism or the pharmaceutical composition of the invention may be provided in encapsulated form, may be used as food supplement or may be incorporated into food products.

The transgenic probiotic microorganism of the invention and the pharmaceutical composition of the invention can be used in treatment of gastro-intestinal inflammatory diseases.

As used herein, the term "treating" refers to reversing, alleviating or inhibiting the progress of a disease, disorder or condition, or one or more symptoms of such disease, disorder or condition, to which such term applies. As used herein, "treating" may also refer to decreasing the probability or incidence of the occurrence of a disease, disorder or condition in a mammal as compared to an untreated control population, or as compared to the same mammal prior to treatment. For example, as used herein, "treating" may refer to preventing a disease, disorder or condition, and may include delaying or preventing the onset of a disease, disorder or condition, or delaying or preventing the symptoms associated with a disease, disorder or condition. As used herein, "treating" may also refer to reducing the severity of a disease, disorder or condition or symptoms associated with such disease, disorder or condition prior to a mammal's affliction with the disease, disorder or condition. Such prevention or reduction of the severity of a disease, disorder or condition prior to affliction relates to the administration of the composition of the present invention, as described herein, to a subject that is not at the time of administration afflicted with the disease, disorder or condition. As used herein "treating" may also refer to preventing the recurrence of a disease, disorder or condition or of one or more symptoms associated with such disease, disorder or condition. The terms "treatment" and "therapeutically," as used herein, refer to the act of treating, as "treating" is defined above.

The transgenic probiotic microorganism and the pharmaceutical composition of the invention are intended for use in treatment of gastro-intestinal inflammatory diseases. Gastro-intestinal inflammatory diseases comprise diseases of the gastro-intestinal tract wherein an inflammatory component is involved like e.g. inflammatory bowel diseases (IBD). The main forms of IBD are Crohn's disease (CD) and ulcerative colitis (UC). Other forms of IBD comprise Collagenous colitis, Lymphocytic colitis, Ischaemic colitis, Diversion colitis, Behçet's disease and Indeterminate colitis.

Preferably, the transgenic probiotic microorganism of the invention and the pharmaceutical composition of the invention are used in treatment of inflammatory bowel diseases. Particularly, the transgenic probiotic microorganism of the invention and the pharmaceutical composition of the invention are used in treatment of Crohn's disease and/or ulcerative colitis.

It has surprisingly found that treatment with transgenic probiotic organisms of the invention leads to improvement of intestinal barrier function. It has been shown that such treatment leads to improved intestinal transepithelial resistance and, thus, is suitable for treatment of intestinal hyperpermeability. Impaired barrier function, impaired transepithelial resistance and intestinal hyperpermeability can be diagnosed by numerous methods, e.g. by determination of translocation of lipopolysaccharides (LPS) across the gut wall of the patient. Thus, the transgenic probiotic organism of the invention is suitable for use in treatment of gastro-intestinal inflammatory diseases and is particularly suitable for treatment of such patient populations which are characterized by impaired intestinal barrier function, impaired intestinal transepithelial resistance, and/or intestinal hyperpermeability.

The transgenic probiotic organism of the invention is preferably used for treatment of gastro-intestinal inflammatory diseases, in particular of a form of inflammatory bowel disease (IBD), wherein patients with impaired intestinal barrier function are treated.

The transgenic probiotic organism of the invention is preferably used for treatment of gastro-intestinal inflammatory diseases, in particular of a form of inflammatory bowel disease (IBD), wherein patients with impaired intestinal transepithelial resistance are treated.

The transgenic probiotic organism of the invention is preferably used for treatment of gastro-intestinal inflammatory diseases, in particular of a form of inflammatory bowel disease (IBD), wherein patients with intestinal hyperpermeability are treated.

According to the present invention, the transgenic probiotic microorganism or the pharmaceutical composition of the invention is administered preferably at an effective amount. An "effective amount" is the dose of the transgenic probiotic microorganism or the pharmaceutical composition that upon administration to a patient yields a measurable therapeutical effect with regard to the disease of interest. In the present invention an effective amount is the dose of the transgenic probiotic microorganism or the pharmaceutical composition that upon administration to a patient yields a therapeutic effect with regard to a gastro-inflammatory disease, like e.g. IBD.

As the current standard treatments for IBD are often ineffective, we have developed a strategy combining the highly effective anti-inflammatory potential of a parasite immunomodulator (AvCystatin) with the beneficial effects of a probiotic bacterium *(Escherichia coli* Nissle 1917). The bacterium is genetically engineered to produce the parasite protein and upon intestinal colonization allows for the direct and prolonged delivery of this potent immune modulator to the inflamed intestine. This excellent, cost-effective new strategy of treating IBD has the potential to support or replace standard treatments.

### FIGURES:

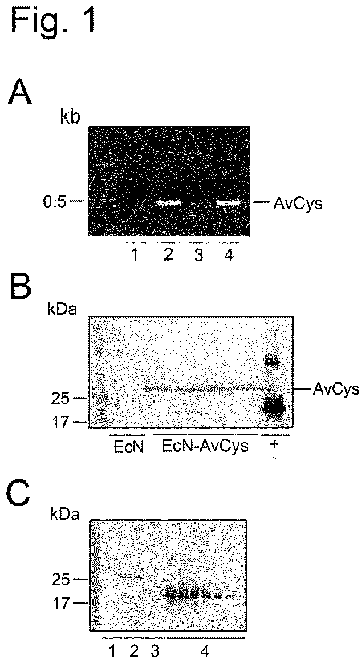

- **Figure 1:**: shows evidence of successful ligation and transformation of *A. viteae* Cystatin encoding gene into an *E. coli* Nissle 1917 carrier and secretion of AvCystatin by transgenic EcN-AvCys. (A) Polymerase Chain Reaction with *A. viteae* Cystatin (AvCys) specific primers successfully amplified a region the AvCyst gene ligated in the modified plasmid (pMU13AvCyst, lane 2) but not in the plasmid lacking the gene insert (pMu13, lane 3). Lane 1: water control, lane 4 postive control. (B, C) Western blot analyses was performed with an AvCys-specific antibody on supernatants of EcN-AvCys and EcN grown in Luria broth medium (B) or 1ml Iscoves's Modified Dulbecco's Medium (IMDM, C), the latter used for porcine and human tissue and cell culture studies. 1: 2 lanes of IMDM controls, 2: 2 lanes of IMDM conditioned by EcN-AvCys, 3: 2 lanes IMDM conditioned by EcN control, 4: 7 lanes loaded with recombinant AvCys (200 - 5ng).

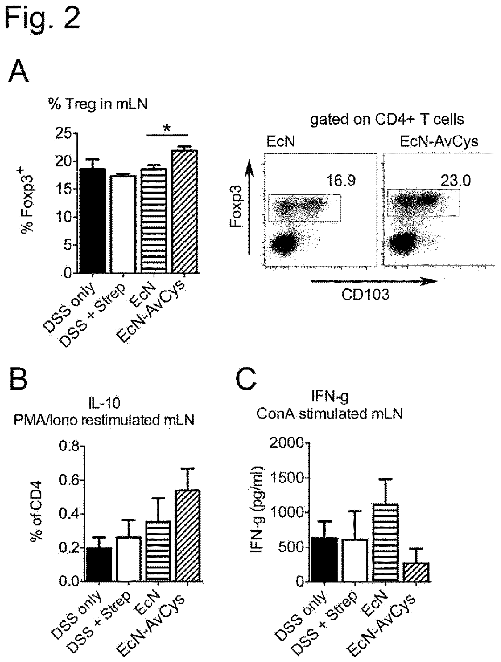

- **Figure 2:**: shows the effect of transgenic EcN-AvCys on a murine model of chronic colitis. **(A)** A significant increase in frequencies of Foxp3+ T cells (Treg) was observed in the EcN-AvCys treated group compared to the group receiving control EcN. **(B, C)** In concanavalin A re-stimulated cells from the gut draining mesenteric lymph nodes (mLN) an increase in regulatory cytokine IL-10 **(B)** and a decrease in pro-inflammatory cytokine IFN-gamma **(C)** was observed in dextran sodium sulphate (DSS) induced colitic mice treated with EcN-AvCys compared to the control groups receiving DSS alone (DSS) or treated with control *E. coli* Nissle (EcN). * p < 0.05.

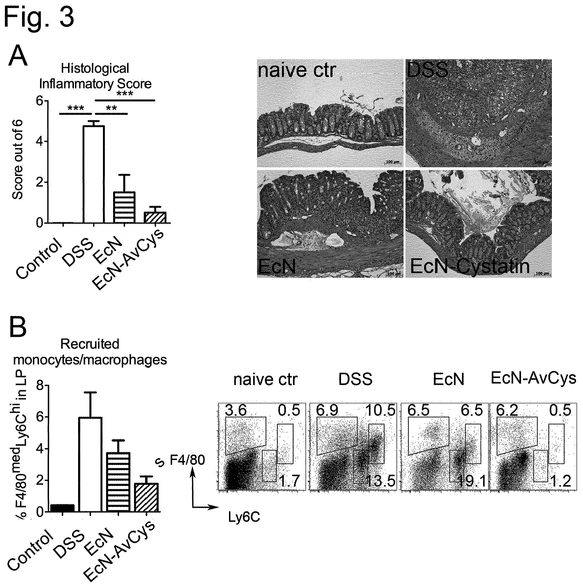

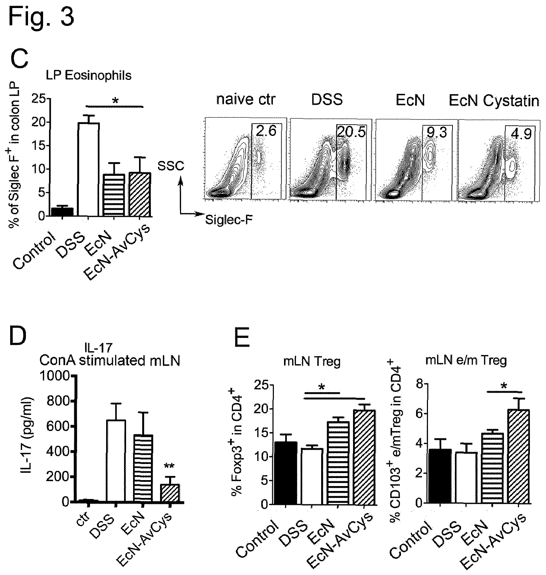

- **Figure 3:**: shows the effect of transgenic EcN-AvCys on a murine model of acute colitis. **(A)** A significant decrease in inflammatory score of colon samples from DSS induced colitic mice treated with EcN-AvCys was observed compared to the control group receiving DSS alone. **(B)** A decrease in newly recruited monocytes/macrophages as determined by flow cytometry with colon lamina propria cells was observed between the EcN-AvCys treated group and DSS controls. **(C)** Infiltration of eospinophils in the lamina propria was reduced in the EcN-AvCys treated group compared to DSS controls. **(D)** Production of the inflammatory cytokine IL-17 by cells isolated from the gut-draining mesenteric lymph nodes in response to stimulation with the mitogen concanavalin A was significantly lower in EcN-AvCys group compared with EcN treated controls. (E) Percentage of Foxp3+ Tregs and CD103+Foxp3+ effector memory (e/m) Tregs in the mesenteric lymph nodes was significantly higher in the EcN-AvCys treated group than in the DSS controls or the EcN treated animals. * p < 0.05, **p< 0.01, ***p< 0.001.

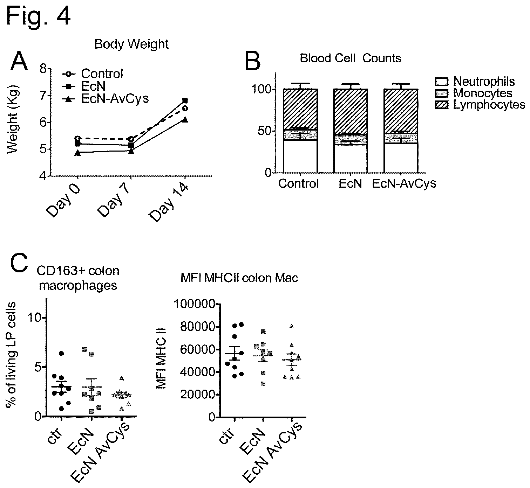

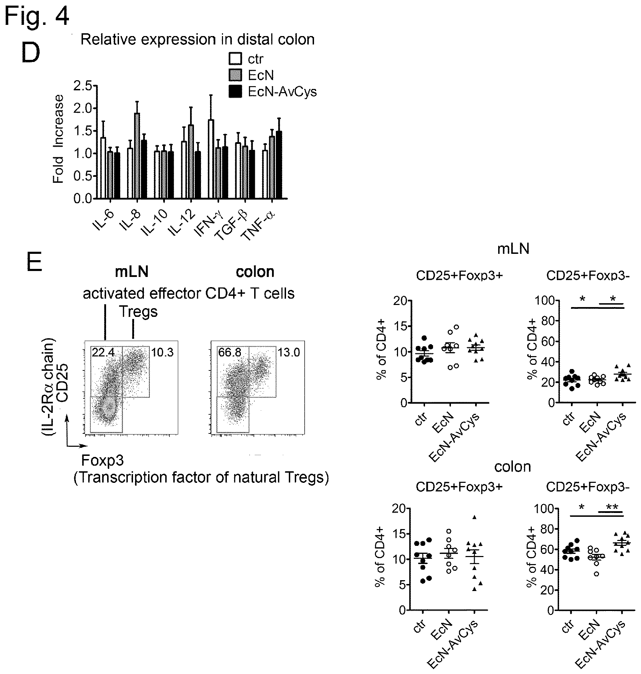

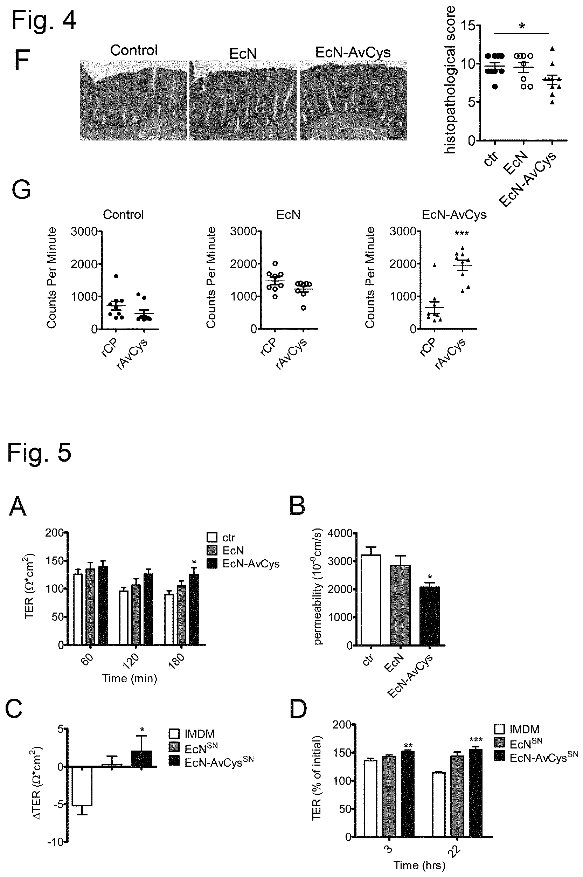

- **Figure 4:**: shows safety and effects of transgenic EcN-AvCys in post-weaning piglets. **(A)** Body weight gain observed for piglets fed EcN-AvCys, EcN or control piglets fed saline alone. **(B)** Between treatment groups there was no significant difference in proportions of cell types counted in blood smears or **(C)** numbers infiltration or activation of monocytes and macrophages as determined by flow cytometric analysis of CD163 and MHCII markers, respectively. **(D)** Expression profiles of pro- and anti-inflammatory cytokines in the colon as determined with RT-PCR were similar in all treatment groups. **(E)** Left: exemplary flow cytometry plots showing the detection of Tregs based on the expression of Foxp3 and CD25 in CD4+ T cells derived from mLN and colon (upper right gates). Frequencies of mLN and colon CD4+CD25+Foxp3+ Tregs did not differ depending on the treatment (center graphs). A significant increase of CD4+CD25+Foxp3- cells was detected in mLN and colon of EcN-AvCys treated pigs (graphs on the right). **(F)** Left: Exemplary hematoxilin & eosin stained cross sections from distal colon used for histopathological scoring of tissue inflammation in post-weaning piglets. Right: Histopathological inflammation score of distal colon detected for the three treatment groups. (G) Proliferative response of mLN leukocytes after 72h incubation with a recombinant control protein (rCP) or recombinant AvCys (rAvCys) as determined by ³H-thymidine incorporation. * p < 0.05, ** p < 0.01.
- **Figure 5:**: shows that treatment with EcN-AvCys and EcN-AvCys supernatant supports epithelial barrier functions. **(A)** Transepithelial resistance (TER) of excised colon tissue from pigs treated with EcN, EcN-AvCys and controls was determined in Ussing chambers at 60, 120 and 180 min after tissue stabilization. **(B)** Colon tissue from euthanized piglets was excised and permeability of the 332Da tracer molecule fluorescein was determined in Ussing chambers. **(C)** Ussing chambers were utilized in order to determine the change in TER across colon tissues excised from untreated, healthy piglets before and after exposure to either IMDM (IMDM, n=7), EcN conditioned IMDM media (EcN^{SN}, n=7) or EcN-AvCys conditioned IMDM media (EcN-AvCys^{SN}, n=7) for 6 h. **(D)** TER of HT-29/B6 cell monolayers exposed to either IMDM (n=6), EcN conditioned IMDM media (n=6) or EcN-AvCys conditioned IMDM media (n=6). * p < 0.05, ** p < 0.01, *** p < 0.001.

### EXAMPLES:

### Materials and methods:

### Construction of the Transgenic Probiotic

A spontaneously streptomycin resistant strain of EcN was gifted to us graciously by Dr. Tobias Oelschläger from the University of Würzburg. The strain also carries a kanamycin resistance cassette on the EcN specific plasmid pMUT2 and has a genetically modified version of the native EcN specific plasmid pMUT1. The modified pMUT1 plasmid, known as pMU13 was designed for functionality as a cloning vector through the addition of a tetracycline resistance cassette, a multiple cloning site and a hemolysin A secretion system. All Nsil cut sites outside of the hlyA gene were deleted to allow for insertion of genes within the hlyA component of the hemolysin A secretion system. This ensures that proteins expressed from the inserted gene will be secreted from the bacteria.

For insertion of AvCystatin into the pMUT13 vector primers were designed to add restriction enzyme, Nsil cut sites to both the 3' and 5' ends of the AvCystatin sequence when amplified (Fw 5'-ACGTATGCATTGGTGCGCTGTGAAGA-3' [SEQ ID NO. 5], Rv 5'-ACGTATGCATTCACTGATGAGAGTACT-3' [SEQ ID NO. 6]). The amplified AvCystatin gene was then inserted into the pMU13 vector at the Nsil cut site and transformed into chemically competent EcN. Successful clones were confirmed with AvCystatin specific primers (Fw 5'-TCGTGTCGACGGTTTTGGTGCGCTGTGAAGAAC-3' [SEQ ID NO. 7], Rv 5'-ACATGCGGCCGCTCACACTGATGAGAGTA-3' [SEQ ID NO. 8]) and western blot analysis of supernatants with AvCystatin specific antibodies.

### Production of EcN and EcN-AvCys supernatant

Cultures of EcN-AvCys and control EcN in Luria broth media containing 20µg/ml tetracycline, 50 µg/ml kanamycin and 50 µg/ml streptomycin were incubated overnight at 37°C with shaking at 200rpm. Cultures were diluted 1:1000 in fresh Iscoves Modified Dulbeccos Media (IMDM) with 4 mM L-glutamine (PAA, Austria) and further incubated at 37 °C with shaking at 200rpm until an OD₆₀₀ = 1.00. Cultures were then centrifuged and supernatant filtered through 0.2µm filters, before being stored at -20 °C. Concentration of AvCys was determined using a direct coating ELISA using a recombinant AvCys standard curve and an anti-rAvCys monoclonal antibody developed in house. Amounts of 12-24ng/ml of rAvCys were specifically detected in three batches of EcN-AvCys conditioned media.

### Animal Experimentation Protocol

Male, 9-11 week old C57BL/6 mice were assigned to one of four groups, a negative control group (Ctr), a positive DSS control group (DSS) an *E. coli* Nissle treated group (EcN) or a transgenic AvCystatin expressing *E. coli* Nissle treated group (EcN-AvCys). Animals in the DSS, EcN and EcN-AvCys group were treated for 7-9 days with 2.5-3.0% DSS in the drinking water. Immediately after the introduction to DSS and every 48hrs thereafter, EcN and EcN-AvCys groups were treated via oral gavage with 100µl of 2x10¹⁰ CFU/ml EcN or transgenic EcN-AvCys in 0.9% saline, respectively. Animals in Ctr and DSS groups were given 100µl of 0.9% saline via oral gavage at the same time points. Animals were euthanized before body weight loss reached 80%. All experiments were in accordance with the German law for the protection of animals and approved by the local authority "Landesamt fur Gesundheit und Soziales".

### EcN/EcN-AvCys verification

Feces from all animals were collected one day before DSS and probiotic administration as well as at dissection. The previously described *E. coli* specific multiplex PCR (Blum-Oehler et al, 2003) was used to determine presence or absence of EcN, while an AvCystatin specific primer set (Fw 5'-TCGTGTCGACGGTTTTGGTGCGCTGTGAAGAAC-3' [SEQ ID NO. 9], Rv 5'-ACATGCGGCCGCTCACACTGATGAGAGTA-3' [SEQ ID NO. 10]) was used to amplify the transgene.

### Quantification of pig blood leukocytes

Fresh venous blood was smeared onto glass slides and stained with Hemacolor Staining Kit (Merck Millipore, Germany). Proportions of monocytes, granulocytes and lymphocytes were counted per 100 leukocytes twice each by two persons and the average of the four counts reported.

### Histopathological analysis

Porcine and murine distal colon sections were fixed in 3.7% formalin, dehydrated using ethanol concentrations increasing from 70-96%, cleared with xylene, embedded in paraffin, mounted and stained with hematoxilin/eosin (H&E) for histopathological scoring according to two systems. The scoring parameters for the DSS-induced colitis model were as follows: inflammation (0: no inflammation; 1: increased number of inflammatory cells in LP; 2: inflammatory cells extending into the submucosa; 3: transmural inflammatory infiltrates) and tissue damage: (0: no mucosal damage; 1: discrete epithelial lesion; 2: erosion or focal ulceration; 3: severe mucosal damage with extended ulcerations extending into bowel wall. Porcine colon cross sections were scored according to the following parameters: infiltration (1: minimal; 2: mild; 3: moderate; 4: severe), degree of infiltration (1: mucosal; 2: mucosal and focal submucosal; 3: mucosal and submucosal; 4: transmural), epithelial surface damage (1: focal denudation; 2: extensive denudation; 3: erosion; 4: ulceration), crypt epithelial damage (1: sporadic crypt abscesses; 2: multiple crypt abscesses) and hyperplasia (1: minimal; 2: mild; 3: moderate; 4: severe).

### In Vitro Cell Culture

Spleen and mesenteric lymph nodes (mLN) were aseptically passed through cell strainers and washed in RPMI 1640 media containing 5% FCS (Biochrom, Germany), 20mM L-glutamine (PAA), 100U/mL penicillin and 100mg/mL streptomycin (PAA). Erythrocytes were removed with lysis buffer (0.01M KHCO₃, 0.155M NH₄Cl, 0.1 mM EDTA, pH 7.5) for 5 min on ice, washed and taken up in RPMI 1640 containing 10% FCS. Cells were counted with a CASY counter (Innovatis, Germany) and 3.5x10⁵ cells /well were plated onto 96 well plates. Cells were stimulated with concanavalin A (2 µg/mL) in a final volume of 200µl. After 48hrs incubation at 37°C with 5% CO₂ supernatants were removed and stored in -20°C until further analysis.

### Cytokine Analysis

Murine specific ELISA antibody pairs for IL-10 (BD Biosciences), IL-17 (R&D Systems) and IFN-γ (eBioscience) were used according to manufacturer recommendations to determine the cytokine levels from the in vitro culture supernatants.

### Quantitative real-time-PCR

Porcine distal colon segments were snap frozen. As per manufacturers protocols, 200mg of distal colon tissue was homogenized using the FastPrep-24 Lysing Matrix Tubes D (MP Biomedical, Germany), RNA was extracted using innuPREP RNA kit (Analytikjena, Germany) and finally cDNA transcribed with the High Capacity RNA-to-cDNA Kit (Applied Biosystems, Germany). Real-Time PCR reactions were conducted in a Mastercycler ep gradient S thermocycler with a Realplex2 detection system (Eppendorf, Germany) using FastStart Universal SYBR Green Master Mix (Roche). Specific primers for swine the swine housekeeping gene RPL19 as well as the cytokines TGF-ß, IL-6 and IL-10 were adapted from Pieper et al. 2012 (J Nutr 142: 661-667). Specific primers for swine IL-8, IL-12, IFN-γ, and TNFα were developed based on the NCBI reference gene sequences (Table 1). The ΔΔCT method was used to determine the fold increase of these cytokines in treatment groups compared to the control group using CT values normalized to a house keeping gene (RPL19).

### Flow Cytometry

Fluorescence labeled anti-murine antibodies for markers Ly-6G (clone RB6-8C5), F4/80 (clone BM8), Ly-6C (clone HK1.4), MHCII (clone M5/114.15.2), CD4 (clone RM4-5), CD25 (clone PC61.5), CD103 (clone 2E7), and FoxP3 (FJK-16S) as well as fixable viability dye and rat IgG isotype controls (eBRG1) were obtained from eBioscience. Anti-murine, fluorescently labeled antibodies for Siglec-F (clone E50-2440) and CD11b (clone M1/70) were obtained from BD Bioscience. Anti FCγRII (clone 24G2) was gratefully received from the Deutsches Rheuma Forschungszentrum Berlin. Porcine leukocytes extracted from mLN and colon LP were stained with the following antibodies: unconjugated αCD25 (clone K231.3B2), unconjugated αSLA class II DR (2E9/13), αMacrophage-FITC (BA4D5), αCD163-PE (2A10/11) and biotinylated sheep αmouse IgG (all AbD Serotec, Germany). αCD4a-PE (74-12-4) was purchased from Southern Biotech (USA). αFoxP3-eFluor450 staining kit (FJK-16S), streptavidin-PE-Cy7 and eFluor 780 fixable viability dye were purchased from eBioscience (USA). Surface staining was performed in PBS, 0.2% BSA. For detection of Foxp3 expression, cells were treated with the fixation/permeabilization buffers from eBioscience according to the manufacturer's instructions. Cells were acquired using an LSRFortessa cell analyzer (BD Biosciences) and analyzed with FloJo software (Treestar, USA).

### Thymidine proliferation assay

Porcine mLN cells were incubated with rAvCys or a recombinant control protein for 72 hrs and then pulsed with 1 µCi of ³H-thymidine (MP Biomedicals, Germany) for 20 h. The proliferative response was analyzed using a beta-counter (PerkinElmer, Germany).

### Electrophysiological studies

Stripped mucosae were mounted into Ussing chambers. These were driven by a multichannel computer-controlled voltage clamp device. The bath solution contained (in mmol/L): 113.6 NaCl, 2.4 Na₂HPO₄, 0.6 NaH₂PO₄, 21 NaHCO₃, 5.4 KCl, 1.2 CaCl₂, 1 MgSO₄, 10 D(+)-glucose, 0.5 β-OH-butyrate and 2.5 glutamine. The solution was gassed and mixed using a bubble lift (95% O₂ and 5% CO₂, pH 7.4). The temperature was kept constant at 37°C. Antibiotics (Azlocillin 50 mg/L and Tobramycin 4 mg/L served to prevent bacterial growth and had no effect on short circuit current (ISC in µA/cm²). Each side of the tissue was perfused with 5 ml of bathing solution and BSA (final concentration of 0.002%) was routinely added and short-circuit current (ISC in µA/cm²) and transmucosal resistance (TER in Ω·cm²) were continuously recorded. Resistance of the bathing solution and electrode offsets were determined and subtracted from raw data before each experiment. Permeability to fluorescein (Sigma, Taufenkirchen, Germany) from mucosal to serosal compartment was determined during a 2 h period (1 to 3 h total time) in Ussing chambers in voltage-clamp mode. Briefly, 100µM of fluorescein was added to the mucosal compartment and serosal concentration was measured as fluorescence using a plate reader (TECAN 200M, Groeding, Austria). Permeabilities were calculated from: (dQ/dt)/(A×Co), where dQ/dt is the cumulative amount of tracer compound appearing in the receiver compartment vs. time, A is the area tissue, and Co is the initial concentration of the tracer in the donor compartment. HT-29/B6 cells, a subclone of the human colon carcinoma cell line HT-29, were cultured in RPMI 1640 (PAA Laboratories GmbH, Pasching, Austria) supplemented with 10% fetal calf serum, and 1% penicillin/streptomycin 37°C in an air atmosphere containing 5% CO₂. For experiments, cells were seeded on Millicell PCF filter supports (effective area 0.6 cm², 3 µm pores, Millicell PCF, Millipore, Schwalbach, Germany). Confluent monolayers were incubated in IMDM, EcN conditioned IMDM or EcN-AvCys conditioned IMDM media from both sides. Transepithelial resistance (TER, Ω·cm²) was determined with an ohmmeter.

### Statistics

The study includes 4 experiments on the effects of EcN-AvCys on murine colitis with 3-5 animals per group and one experiment with pigs with group sizes of 8-10. Groups were compared with one-way and two-way ANOVA as well as Mann-Whitney U test using GraphPad Prism software (San Diego, USA). Data are reported as means±SEM and differences were deemed significant if p<0.05.

### Results:

A native EcN-specific plasmid was modified to include a multiple cloning site for ease of gene insertion, an antibiotic resistance gene for selection purposes and a secretion system to allow inserted gene products to be secreted by the bacteria. The AvCystatin encoding gene was amplified using primers designed to add restriction enzyme cut sites to the 3' and 5' ends of the gene sequence. Using restriction enzyme digestion and ligation, the amplicon was then inserted in frame into the vector at a site that allows both expression and secretion of the functional protein upon transformation of the vector into EcN.

EcN-AvCys has been verified by both molecular, PCR based determination of the cystatin gene in extracted plasmid DNA (Fig. 1A), as well as by Western blot analysis of supernatant of bacterial cultures (Fig. 1B, C). The expression and secretion of AvCys by transgenic EcN-AvCys was studied through western blot analysis of EcN-AvCys culture supernatant (EcN-AvCys^{SN}) with a monoclonal anti-AvCys antibody. Resulting data showed that EcN produced significant amounts of recombinant AvCys in different cell culture media when cultures were grown to a concentration of 3x10⁸ CFU EcN/ml **(****Fig. 1** **B, C).** The concentration of AvCys in EcN-AvCys^{SN} as determined by ELISA varied between 12-24ng/ml when three seperate batches were analyzed.

### The effect of a single application of nematode cystatin-expressing, transgenic EcN on a murine model of chronic colitis.

Adult male C57BL/6 mice were administered 3 cylces of 2.5% dextran sodium sulphate (DSS, a chemical disrupting the epithelial barrier of the intestine and thus leading to a colitic response) in the drinking water for 7 days with rest periods of 10 days between cycles. On day 38, the first day of the last DSS cycle, either 2x10⁹ CFU EcN-AvCys, 2x10⁹ CFU control EcN, or saline was administered via oral administration. Colonization was supported through application of streptomycin in the drinking water on day 37, one day before bacterial administration until sacrifice on day 45. While histological inflammatory scores were not significantly improved (not shown) in the EcN-AvCys groups compared to the EcN or saline control groups a significant increase in Foxp3+ regulatory T cells (Fig. 2A) and a trend towards increased production of regulatory cytokine IL-10 (Fig. 2B) was observed upon mitogen-driven stimulation of lymphocytes from the gut draining mesenteric lymph nodes. Likewise, a trend towards decreased production of the inflammatory cytokine IFN-gamma was observed (Fig. 2C). This study, performed in a very harsh, challenging colitis model, shows the potential of EcN-AvCys in ameliorating chronic colitis, as seen by the support for Tregs and regulatory cytokine expression and the suppression of inflammatory cytokine production.

### The effect of multiple applications of nematode cystatin-expressing, transgenic EcN on a murine model of acute colitis.

Adult male C57BL/6 mice were administered 1 cylce of 3% DSS in the drinking water for 8 days. Either 2x10⁹ CFU EcN-AvCys, 2x10⁹ CFU EcN or saline was administered via oral administration every 2 days throughout the DSS application. Animals were sacrificed at day 8. There was a significant decrease in histological inflammatory score in animals treated with EcN or EcN-AvCys compared with saline treated controls (Fig. 3A). The lowest inflammatory scores were observed in mice receiving EcN-AvCys (Fig. 3A). A reduction in recruitment and activation of monocytes/macrophages, indicators of inflammation, to the colon lamina propria was observed in EcN-AvCys treated groups compared to saline control animals (Fig. 3B). Likewise a decrease in colon eosinophilia (Fig. 3C) and inflammatory cytokine (IL-17) production by cells from the gut-draining mesenteric lymph nodes (Fig. 3D) was also observed in EcN-AvCys treated mice compared with the controls. Again, Foxp3+ and activated CD103+ regulatory T cells were significantly increased in the mesenteric lymph nodes of the EcN-AvCys treated group compared with EcN alone or saline treated controls (Fig. 3E). This trial demonstrates the potential of EcN-AvCys in the attenuation of inflammation in acute colitis as demonstrated through the induction of regulatory cells and the down-regulation of inflammatory cell infiltration and pro-inflammatory cytokine expression.

### Safety of transgenic parasite EcN-AvCys in a porcine model and amelioration of post-weaning gut inflammation

Post weaning male piglets were given either 2x10¹⁰ EcN-AvCys, 2x10¹⁰ EcN or saline control via oral syringe every 2nd day for 14 days. Animals were euthanized at day 14. No significant differences in weight gain (Fig. 4A), blood cell populations (Fig. 4B), frequencies and activation of colon macrophages (Fig. 4C), inflammatory and regulatory cytokine expression in the colon (Fig. 4D) or percentage of CD25+FoxP3+ regulatory T cells (Fig. 4E) were observed between treatment groups. However, CD25+Foxp3-CD4+ T cells, a cell subset recently attributed with a regulatory function in human patients (Triplett et al. 2012, Eur J Immunol 42: 1893-1905) significantly increased in mLN and colon of piglets receiving EcN-AvCys (Fig. 4E). This study demonstrates the safety of EcN-AvCys application in healthy monogastrics as a therapeutic for gastrointestinal inflammatory diseases.

As weaning is associated with an inflammatory response in the intestine of piglets, we scored distal colon cross sections histopathologically. Indeed pigs from the control groups showed moderate signs of inflammation (Fig. 4F). Recipients of EcN-AvCys had a significantly reduced inflammatory score compared to the untreated control group (Fig. 4F), pointing towards a beneficial influence of the transgenic bacteria secreting a helminth immunomodulator.

In order to ascertain that AvCys was produced by EcN-AvCys and was recognized by the immune system, we isolated cells from the mLN and stimulated them with rAvCys or a recombinant control protein expressed and purified accordingly. Only mLN cells from EcN-AvCys treated pigs proliferated specifically in response to rAvCys compared to the recombinant control protein (Fig. 4G).

Collectively these data suggest that the application of EcN-AvCys to piglets in the post-weaning period was well tolerated. Administration of EcN-AvCys did not lead to changes in body weight development or systemic and local immune parameters (other than a local increase in CD4+CD25+ T cells). Interestingly, we found significantly reduced signs of post-weaning associated intestinal inflammatory reactions in recipients of the transgenic probiotic and this was associated with an increase of CD4+CD25+ T cells in mLN and colon after treatment with EcN-AvCys.

### Transgenic probiotic improves intestinal barrier function

As EcN was reported to support intestinal barrier function we tested whether the additional secretion of AvCys affected this feature of the probiotic. Proximal colon tissue from pigs fed EcN-AvCys, EcN or saline alone were analyzed in Ussing chambers in order to determine the transepithelial resistance (TER) and paracellular passage of the small tracer molecule fluorescein (332 Da). The EcN-AvCys group showed significantly higher TER to small inorganic ions than the saline control group at 180min, whereas tissue from piglets inoculated with EcN alone showed no significant improvement compared to controls (Fig. 5A). A significant decrease in paracellular passage of a larger organic solute (fluorescein) was observed in proximal colon tissue from EcN-AvCys treated piglets, but not in EcN inoculated piglets, when compared to the saline control group (Fig. 5B). These data suggest that EcN-AvCys is able to improve epithelial barrier function in vivo in a manner that is unaffected by EcN treatment alone.

Next we sought to ascertain if prolonged contact of intestinal tissue with EcN-AvCys in vivo was necessary for the supportive effects on epithelial barrier functions or whether exposure to EcN-AvCys culture supernatant (EcN-AvCys^{SN}) could induce similar effects. We determined the difference in transepithelial resistance before and after exposure of colon tissue from untreated weaned piglets to either EcN-AvCys^{SN}, EcN^{SN} or IMDM. Remarkably, the relatively brief contact of colonic tissue with EcN-AvCys^{SN} also significantly increased TER compared to the control group, the latter showing a decline in TER after incubation with IMDM for 6h (Fig 5C). Contact with EcN^{SN} showed an intermediate effect, preventing the decline on TER measured with IMDM controls (Fig 5C). To evaluate the effects of EcN-AvCys^{SN} on pure cultures of epithelial cells we further analyzed its influence on the human colon epithelial cell line HT-29/B6. Monolayers were incubated with EcN-AvCys^{SN}, EcN^{SN} or IMDM. A significant increase in TER was observed in cells incubated for 3 hrs with EcN-AvCys^{SN} and, to an even greater extent at 22 h when compared to IMDM controls (Fig. 5D). Taken together these data indicate that EcN-AvCys has a strong potential to enhance intestinal epithelial barrier functions in vivo and that remarkably, bacterial culture supernatants are also able to enhance barrier function after only relatively short contact with either porcine colon tissue or human epithelial cells in vitro.

## Claims

1. Transgenic probiotic microorganism genetically engineered to express and secrete nematode derived cystatin, wherein the probiotic microorganism is *Escherichia coli* strain Nissle 1917; and wherein the nematode derived cystatin comprises or consists of:
- an amino acid sequence with SEQ ID NO. 1, 2 or 3; or
- an amino acid sequence that is at least 70% identical, preferably at least 80% identical, more preferably at least 90% identical and most preferably at least 99% identical to an amino acid sequence with SEQ ID NO. 1, 2 or 3.

2. Transgenic probiotic microorganism of claim 1, wherein the nematode derived cystatin comprises or consists of:
- an amino acid sequence with SEQ ID NO. 1; or
- an amino acid sequence that is at least 70% identical, preferably at least 80% identical, more preferably at least 90% identical and most preferably at least 99% identical to an amino acid sequence with SEQ ID NO. 1.

3. Pharmaceutical composition comprising a transgenic probiotic microorganism of one of claims 1 to 2 and at least one pharmaceutically acceptable excipient.

4. Transgenic probiotic microorganism of one of claims 1 to 2 or pharmaceutical composition of claim 3 for use in treatment of gastro-intestinal inflammatory diseases.

5. Transgenic probiotic microorganism of one of claims 1 to 2 or pharmaceutical composition of claim 3 for use in treatment of inflammatory bowel diseases.

6. Transgenic probiotic microorganism of one of claims 1 to 2 or pharmaceutical composition of claim 3 for use in treatment of Crohn's disease and/or ulcerative colitis.

7. The transgenic probiotic organism for use of one of claims 4 to 6, wherein patients with impaired intestinal barrier function are treated.

8. The transgenic probiotic organism for use of one of claims 4 to 6, wherein patients with impaired intestinal transepithelial resistance are treated.

9. The transgenic probiotic organism for use of one of claims 4 to 6, wherein patients with intestinal hyperpermeability are treated.

## Patentansprüche

1. Transgener probiotischer Mikroorganismus, der gentechnisch verändert ist, um von Nematoden stammendes Cystatin zu exprimieren und zu sekretieren, wobei der probiotische Mikroorganismus *Escherichia coli* Stamm Nissle 1917 ist; und wobei das von Nematoden stammende Cystatin umfasst oder besteht aus:
- eine(r) Aminosäuresequenz mit der SEQ ID NR. 1, 2 oder 3; oder
- eine(r) Aminosäuresequenz, die zu wenigstens 70 % identisch, vorzugsweise zu wenigstens 80 % identisch, besonders bevorzugt zu wenigstens 90 % identisch und ganz besonders bevorzugt zu wenigstens 99 % identisch mit einer Aminosäuresequenz mit der SEQ ID NR. 1, 2 oder 3 ist.

2. Transgener probiotischer Mikroorganismus nach Anspruch 1, wobei das von Nematoden stammende Cystatin umfasst oder besteht aus:
- eine(r) Aminosäuresequenz mit der SEQ ID NR. 1; oder
- eine(r) Aminosäuresequenz, die zu wenigstens 70 % identisch, vorzugsweise zu wenigstens 80 % identisch, besonders bevorzugt zu wenigstens 90 % identisch und ganz besonders bevorzugt zu wenigstens 99 % identisch mit einer Aminosäuresequenz mit der SEQ ID NR. 1 ist.

3. Pharmazeutische Zusammensetzung, die einen transgenen probiotischen Mikroorganismus nach einem der Ansprüche 1 bis 2 und wenigstens einen pharmazeutisch annehmbaren Hilfsstoff umfasst.

4. Transgener probiotischer Mikroorganismus nach einem der Ansprüche 1 bis 2 oder pharmazeutische Zusammensetzung nach Anspruch 3 zur Verwendung bei der Behandlung entzündlicher Erkrankungen des Magen-Darm-Trakts.

5. Transgener probiotischer Mikroorganismus nach einem der Ansprüche 1 bis 2 oder pharmazeutische Zusammensetzung nach Anspruch 3 zur Verwendung bei der Behandlung entzündlicher Darmerkrankungen.

6. Transgener probiotischer Mikroorganismus nach einem der Ansprüche 1 bis 2 oder pharmazeutische Zusammensetzung nach Anspruch 3 zur Verwendung bei der Behandlung von Morbus Crohn und/oder Colitis ulcerosa.

7. Transgener probiotischer Organismus zur Verwendung nach einem der Ansprüche 4 bis 6, wobei Patienten mit gestörter intestinaler Barrierefunktion behandelt werden.

8. Transgener probiotischer Organismus zur Verwendung nach einem der Ansprüche 4 bis 6, wobei Patienten mit gestörtem intestinalem transepithelialem Widerstand behandelt werden.

9. Transgener probiotischer Organismus zur Verwendung nach einem der Ansprüche 4 bis 6, wobei Patienten mit intestinaler Hyperpermeabilität behandelt werden.

## Revendications

1. Microorganisme probiotique transgénique génétiquement modifié pour exprimer et sécréter la crystatine dérivée d'un nématode, où le microorganisme probiotique est *Escherichia coli* Nissle 1917 ; et où la crystatine dérivée d'un nématode comprend, ou consiste en :
- une séquence d'acide aminé représentée par SEQ ID NO. 1, 2 ou 3 ; ou
- une séquence d'acide aminé au moins à 70 % identique, avantageusement au moins à 80 % identique, préférentiellement au moins à 90 % identique et tout particulièrement au moins 99% identique à une séquence d'acide aminé représentée par SEQ ID NO. 1, 2 ou 3.

2. Microorganisme probiotique transgénique selon la revendication 1, où la crystatine dérivée d'un nématode comprend, ou consiste en :
- une séquence d'acide aminé représentée par SEQ ID NO. 1 ; ou
- une séquence d'acide aminé au moins à 70 % identique, avantageusement au moins à 80 % identique, préférentiellement au moins à 90 % identique et tout particulièrement au moins à 99 % identique à une séquence d'acide aminé représentée par SEQ ID NO. 1.

3. Composition pharmaceutique comprenant un microorganisme probiotique transgénique selon la revendication 1 ou la revendication 2 et au moins un excipient pharmaceutiquement acceptable.

4. Microorganisme probiotique transgénique selon la revendication 1 ou la revendication 2 ou composition pharmaceutique selon la revendication 3, utilisable pour le traitement de troubles inflammatoires gastro-intestinaux.

5. Microorganisme probiotique transgénique selon la revendication 1 ou la revendication 2 ou composition pharmaceutique selon la revendication 3, utilisable pour le traitement de troubles inflammatoires de l'intestin.

6. Microorganisme probiotique transgénique selon la revendication 1 ou la revendication 2 ou composition pharmaceutique selon la revendication 3, utilisable pour le traitement de la maladie de Crohn et/ou de la colite ulcéreuse.

7. Organisme probiotique transgénique utilisable selon l'une des revendications 4 à 6, permettant le traitement de patients souffrant de troubles de la fonction de barrière intestinale.

8. Organisme probiotique transgénique utilisable selon l'une des revendications 4 à 6, permettant le traitement de patients souffrant de troubles de la résistance transépithéliale intestinale.

9. Organisme probiotique transgénique utilisable selon l'une des revendications 4 à 6, permettant le traitement de patients souffrant d'hyperperméabilité intestinale.


### SEQUENCE LISTING

<110> Humboldt-Universitat zu Berlin Freie Universitat Berlin
<120> Probiotic bacteria as carrier for a helminth-derived immunomodulator for the treatment of inflammatory disorders
<130> P09932WO
<150> EP12183268
   <151> 2012-09-06
<160> 10
<170> PatentIn version 3.5
<210> 1
   <211> 157
   <212> PRT
   <213> Acanthocheilonema viteae
<400> 1
   Met Met Leu Ser Ile Lys Glu Asp Gly Leu Leu Val Val Leu Leu Leu
   1               5                   10                  15
   Ser Phe Gly Val Thr Thr Val Leu Val Arg Cys Glu Glu Pro Ala Asn
               20                  25                  30
   Met Glu Ser Glu Val Gln Ala Pro Asn Leu Leu Gly Gly Trp Gln Glu
           35                  40                  45
   Arg Asn Pro Glu Glu Lys Glu Ile Gln Asp Leu Leu Pro Lys Val Leu
       50                  55                  60
   Ile Lys Leu Asn Gln Leu Ser Asn Val Glu Tyr His Leu Met Pro Ile
   65                  70                  75                  80
   Lys Leu Leu Lys Val Ser Ser Gln Val Val Ala Gly Leu Arg Tyr Lys
                   85                  90                  95
   Met Glu Ile Gln Val Ala Gln Ser Glu Cys Lys Lys Ser Ser Gly Glu
               100                 105                 110
   Glu Val Asn Leu Lys Thr Cys Lys Arg Leu Glu Gly His Pro Asp Gln
           115                 120                 125
   Ile Ile Thr Leu Glu Ala Trp Glu Lys Ser Trp Glu Asn Phe Leu Gln
       130                 135                 140
   Val Lys Ile Leu Glu Lys Lys Glu Val Leu Ser Ser Val
   145                 150                 155
<210> 2
   <211> 162
   <212> PRT
   <213> Onchocerca volvulus
<400> 2
   Met Leu Thr Ile Lys Asp Gly Thr Leu Leu Ile His Leu Leu Leu Phe
   1               5                   10                  15
   Ser Val Val Ala Leu Val Gln Leu Gln Gly Ala Lys Ser Ala Arg Ala
               20                  25                  30
   Lys Asn Pro Ser Lys Met Glu Ser Lys Thr Gly Glu Asn Gln Asp Arg
           35                  40                  45
   Pro Val Leu Leu Gly Gly Trp Glu Asp Arg Asp Pro Lys Asp Glu Glu
       50                  55                  60
   Ile Leu Glu Leu Leu Pro Ser Ile Leu Met Lys Val Asn Glu Gln Ser
   65                  70                  75                  80
   Asn Asp Glu Tyr His Leu Met Pro Ile Lys Leu Leu Lys Val Ser Ser
                   85                  90                  95
   Gln Val Val Ala Gly Val Lys Tyr Lys Met Asp Val Gln Val Ala Arg
               100                 105                 110
   Ser Gln Cys Lys Lys Ser Ser Asn Glu Lys Val Asp Leu Thr Lys Cys
           115                 120                 125
   Lys Lys Leu Glu Gly His Pro Glu Lys Val Met Thr Leu Glu Val Trp
       130                 135                 140
   Glu Lys Pro Trp Glu Asn Phe Met Arg Val Glu Ile Leu Gly Thr Lys
   145                 150                 155                 160
   Glu Val
<210> 3
   <211> 161
   <212> PRT
   <213> Brugia malayi
<400> 3
   Met Met Ser Thr Met Ser Ile Lys Glu Gly Leu Leu Val Ile Leu Leu
   1               5                   10                  15
   Ser Leu Phe Leu Phe Asp Thr Thr Ala Leu Ile His Arg Arg Glu Ile
               20                  25                  30
   Pro His Met Glu Ser Lys Gly Gln Met Gln Arg Gly Gln Val Leu Leu
           35                  40                  45
   Gly Gly Trp Gln Glu Arg Ser Pro Glu Asp Asn Glu Ile Leu Glu Leu
       50                  55                  60
   Leu Pro Ser Val Leu Thr Lys Val Asn Gln Gln Ser Asn Asp Glu Tyr
   65                  70                  75                  80
   His Leu Met Pro Ile Lys Leu Leu Lys Val Ser Ser Gln Val Val Ala
                   85                  90                  95
   Gly Val Lys Tyr Lys Met Glu Val Gln Val Ala Arg Ser Glu Cys Lys
               100                 105                 110
   Lys Ser Ala Ser Glu Gln Val Asn Leu Lys Thr Cys Lys Lys Leu Glu
           115                 120                 125
   Gly His Pro Asp Gln Val Met Thr Leu Glu Val Trp Glu Lys Pro Trp
       130                 135                 140
   Glu Asp Phe Leu Gln Val Asn Ile Leu Glu Thr Lys Val Leu Ser Ser
   145                 150                 155                 160
   Val
<210> 4
   <211> 687
   <212> DNA
   <213> Acanthocheilonema viteae
<400> 4
   aataaacaaa gctaacgacg atatgatgtt gtcaataaag gaggatggat tgttggtggt      60
   acttttattg tcgttcggtg tgacgacagt tttggtgcgc tgtgaagaac ccgcaaatat     120
   ggaatctgag gtacaagcgc ccaatttatt gggaggatgg caggaacgca atccggaaga     180
   gaaagaaata caggacctgt tgccaaaggt attaattaaa ctaaatcagc tgtcaaacgt     240
   ggagtaccac ctaatgccaa tcaaattact gaaagtttca tctcaagttg tggctggttt     300
   gagatacaag atggaaatac aggttgctca atcagaatgc aaaaaaagtt caggcgagga     360
   agttaatctg aaaacatgta aaagattgga aggacatccg gatcagatta tcacgttgga     420
   ggcatgggag aaatcatggg aaaatttttt gcaagtcaaa attctggaaa aaaaagaagt     480
   actctcatca gtgtgattct ttttgcaagt ctcacttaac gttatccact gtgctttgta     540
   gtatttttat tgtttaatgc ttacgatttt ttttaaaatg cattttgtaa ttttatcaaa     600
   atcaaaatgg tattgtaatt ggtgcaaaaa atatttaaag ttgataatgc caatattcca     660
   gcttataatt gttaaagtac attgtga                                         687
<210> 5
   <211> 26
   <212> DNA
   <213> artificial
<220>
   <223> primer AvCystatin
<400> 5
   acgtatgcat tggtgcgctg tgaaga 26
<210> 6
   <211> 27
   <212> DNA
   <213> artificial
<220>
   <223> primer AvCystatin
<400> 6
   acgtatgcat tcactgatga gagtact 27
<210> 7
   <211> 33
   <212> DNA
   <213> artificial
<220>
   <223> primer AvCystatin
<400> 7
   tcgtgtcgac ggttttggtg cgctgtgaag aac 33
<210> 8
   <211> 29
   <212> DNA
   <213> artificial
<220>
   <223> primer AvCystatin
<400> 8
   acatgcggcc gctcacactg atgagagta 29
<210> 9
   <211> 33
   <212> DNA
   <213> artificial
<220>
   <223> primer AvCystatin
<400> 9
   tcgtgtcgac ggttttggtg cgctgtgaag aac 33
<210> 10
   <211> 29
   <212> DNA
   <213> artificial
<220>
   <223> primer AvCystatin
<400> 10
   acatgcggcc gctcacactg atgagagta 29